# FASCICULE DE BREVET EUROPEEN

(11) **EP 1 178 778 B1**
(45) Date de publication et mention de la délivrance du brevet: **01.03.2006**
(21) Numéro de dépôt: 00929620.3
(22) Date de dépôt: 17.05.2000
(51) Int. Cl.: A61K 9/16, A61K 9/50, A61K 36/00

(54) **GRANULES CONTENANT UNE SUBSTANCE VEGETALE ET LEUR PROCEDE DE PREPARATION**
PFLANZENMATERIAL ENTHALTENDES GRANULAT UND VERFAHREN ZUR HERSTELLUNG
GRANULES CONTAINING A PLANT SUBSTANCE AND METHOD FOR PRODUCING THE SAME

(30) Priorité: 17.05.1999 US 312485
(43) Date de publication de la demande: 13.02.2002
(73) Titulaire: D.B.F., 92210 Saint-Cloud (FR)
(72) Inventeur: DEBREGEAS, Patrice, F-75007 Paris (FR); LEDUC, Gérard, F-45330 Malesherbes (FR); BERNABE, Domingo, F-95330 Domont (FR)
(74) Mandataire: Ahner, Francis
(86) Numéro de dépôt international: PCT/FR2000/001316
(87) Numéro de publication internationale: WO 2000/069414

(56) Documents cités:
- EP-A- 0 290 299
- EP-A- 0 687 466
- WO-A-98/16111
- DE-A- 2 458 112
- FR-A- 1 443 063
- FR-A- 2 790 668

## Description

La présente invention a pour objet une nouvelle formulation sous la forme de granules contenant une substance végétale ainsi que son procédé de préparation.

Plus précisément, la présente invention concerne des granules contenant au moins une substance végétale et comprenant chacun un noyau neutre enrobé d'une couche contenant ladite substance végétale associé à un excipient pharmaceutiquement acceptable.

Les formulations contenant des substances végétales déjà décrites dans l'art antérieur sont sous la forme de poudres, de granules, de comprimés ou de solutions buvables.

Le problème majeur des formulations sous la forme de poudre est que la poudre végétale doit être mélangée à des excipients eux aussi sous forme de poudre. L'on obtient alors un mélange de poudres peu homogène et difficilement reproductible.

De plus, les poudres sont très hygroscopiques et elles pompent donc l'humidité des granules et de la gélule, qui devient cassante. Ceci pose des problèmes de stabilité, et le dosage dans la gélule n'est pas homogène.

Ce problème est résolu dans le cadre de la présente invention, par application d'une substance végétale sous forme sèche ou liquide sur des micrograins neutres : dans ce cas, il n'y a pas de mélange de poudre mais seulement application de substances sèches ou liquides sur des granules neutres.

Les granules selon l'invention présentent l'avantage d'être plus faciles à conditionner en gélules que des poudres, d'être plus stables au stockage que les formulations de l'art antérieur et de permettre un dosage reproductible.

Quant aux comprimés, ils présentent les mêmes problèmes que les poudres. Par ailleurs, les extraits de plantes ne sont pas toujours compressibles et les agents de compression ne sont pas toujours autorisés dans l'industrie alimentaire.

Enfin, les formes fluides buvables sont souvent amères et nauséabondes si bien qu'il faut ajouter des édulcorants et des stabilisants. En outre, les formes fluides buvables peuvent présenter une instabilité physique et chimique au cours de la conservation, une faible teneur en constituants végétaux caractéristiques, et contiennent fréquemment de l'alcool éthylique en quantité plus ou moins importante ce qui n'est généralement pas souhaité pour l'administration par voie orale de produits médicamenteux.

La forme multiparticulaire de la formulation de l'invention permet d'obtenir un profil de libération régulier et reproductible.

En outre, les granules de l'invention qui comportent chacun une couche de substance végétale montée sur un noyau neutre peuvent être enrobés d'une couche externe afin de modifier leurs propriétés. La couche externe comprend par exemple un polymère entérique, un polymère destiné à prolonger la libération de la substance végétale ou un polymère destiné à masquer le goût ou l'odeur de la substance végétale.

La formulation selon l'invention présente l'avantage d'être stable au cours du stockage, de présenter une biodisponibilité améliorée, et de pouvoir intégrer des doses élevées de substance végétale.

FR 2 721 512 décrit un procédé de préparation de granules par extrusion-sphéronisation à partir d'un polymère aux propriétés absorbantes et adsorbantes. Le polymère est pulvérisé avec un extrait végétal fluide hydroalcoolique.

Le polymère, synthétique ou naturel, est éventuellement associé à des substances auxiliaires, comme le lactose ou la PVP, qui permettent de moduler la porosité des sphéroïdes et leur vitesse de dissolution.

La technique d'extrusion-sphéronisation présente de nombreux inconvénients : elle nécessite l'adjonction d'une quantité d'eau au moins égale à la quantité des excipients, les granules obtenus par cette technique présentent des taux d'humidité élevés et leur séchage est très long. En outre, le procédé décrit dans FR 2 721 512 utilise des poudres.

FR 2 616 068 décrit un procédé qui consiste à granuler un extrait végétal sec ou mou avec de la méthylcellulose ou de la silice.

FR 2 682 874 décrit un procédé de préparation d'un extrait de principe actif sous forme sèche à partir d'un extrait fluide, qui consiste à adsorber une solution hydroalcoolique du principe actif sur des grains poreux de cellulose ou de silice. Les grains ont une granulométrie de l'ordre du micron. Ces grains sont ensuite adsorbés sur des granules poreuses de 0,1 à 0,5 mm de diamètre, qui sont par exemple constituées de sucre.

FR 2 737 134 décrit un procédé qui consiste à enrober des noyaux, de diamètre inférieur à 0,01 mm, constitués de maltisorb ou d'un mélange bicarbonate de sodium/citrate, avec un composé sous forme de poudre et un composé en solution. Le composé en solution est une huile essentielle et/ou un extrait fluide concentré de plante.

La présente invention a pour objet des granules qui remédient aux inconvénients des formulations de l'art antérieur. Ces granules contenant au moins une substance végétale sont caractérisés en ce qu'ils comprennent chacun un noyau neutre de granulométrie comprise entre 0,2 et 4 mm enrobé d'une couche contenant la substance végétale associée à un excipient pharmaceutiquement acceptable.

La substance végétale peut être issue des plantes choisies parmi l'ail, l'échinacée, le ginkgo biloba, le ginseng, l'harpagophytum, le kawa kawa, le millepertuis, le thé vert, la valériane, la vigne rouge, l'artichaut, l'aubépine, la bardane, le bouleau, la bourdaine, le cassis, le chardon Marie, le fucus, l'hamamelis, le marron d'Inde, la mélisse, l'orthosiphon, la passiflore, le pissenlit, la prêle, la reine des prés, la sauge, la spiruline, le boldo, l'ortie, le palmier d'Amérique du Nord et leurs mélanges.

Le noyau neutre est constitué d'une substance choisie parmi le sucre, l'amidon, le mannitol, le sorbitol, le xylitol, la cellulose, le talc et leurs mélanges.

Les noyaux neutres peuvent être également constitués d'un coeur d'amidon/saccharose dans les proportions massiques 20/80 enrobé de 80 % en poids d'amidon. Dans de tels noyaux neutres, la proportion massique de sucre est avantageusement inférieure à 20 %.

La couche contenant la substance végétale contient comme liant la polyvinylpyrrolidone (PVP).

Les granules selon l'invention peuvent être constitués d'un noyau neutre enrobé d'une couche contenant la substance végétale, elle même enrobée d'une couche externe destinée à masquer le goût et/ou l'odeur de la substance végétale, à retarder sa libération ou à contrôler sa libération.

Lorsque la couche externe est destinée à contrôler la libération de la substance végétale elle contient avantageusement de la gomme laque, de la PVP, un copolymère de l'acide méthacrylique (Eudragit®) ou une dispersion colloïdale d'éthylcellulose (Aquacoat®) avec un plastifiant.

Lorsque la couche externe est destinée à retarder la libération de la substance végétale, elle contient un copolymère de l'acide méthacrylique, de la gomme laque ou une dispersion colloïdale d'éthylcellulose (Aquacoat®) avec un plastifiant.

Comme polymère destiné à masquer le goût et/ou l'odeur de la substance végétale, on pourra utiliser un copolymère de l'acide méthacrylique (l'Eudragit E 100®), une dispersion de polyacrylate (l'Eudragit NE 30D®) ou l'hydroxypropylméthylcellulose (Pharmacoat®).

On peut utiliser comme polymère entérique la gomme laque en pulvérisant une solution alcoolique à 10 % en poids de gomme laque. A des concentrations supérieures, comprises entre 20 et 40 %, la gomme laque remplit la fonction de polymère à libération retardée. Dans les granules, la teneur en substance végétale est comprise entre 0,1 mg/g et 750 mg/g.

La présente invention concerne en particulier des granules d'ail à odeur et goût masqués, des granules de gingko biloba une prise jour, des granules de ginseng à libération prolongée, des granules d'Harpagophytum entériques, des granules de thé vert à libération prolongée, des granules d'orthosiphon à libération prolongée, des granules de valériane à goût et odeur masqués et des granules de millepertuis à libération prolongée.

La présente invention concerne également un procédé de préparation des granules décrits précédemment. Le procédé selon l'invention permet une meilleure reproductibilité du dosage. Les granules selon l'invention peuvent contenir plusieurs substances végétales utilisées sous la forme de poudre de plantes, d'extraits secs, mous ou fluides de plantes ou de leurs mélanges.

Selon la définition donnée dans la Pharmacopée, les extraits végétaux sont des préparations concentrées, liquides, solides ou de consistance intermédiaire, généralement obtenues à partir de matières premières végétales séchées. Pour certaines préparations, les matières à extraire peuvent subir un traitement préalable tel que l'inactivation d'enzymes, le broyage ou le dégraissage.

Les extraits fluides sont des préparations liquides dont, en général, une partie en masse ou en volume correspond à une partie en masse de matière première séchée. Ces préparations sont ajustées, si nécessaire, de façon à répondre aux exigences de la teneur en solvants, en constituants ou en résidu sec.

Les extraits mous sont des préparations de consistance intermédiaire entre les extraits fluides et les extraits secs. Les extraits mous sont préparés par évaporation partielle du solvant ayant servi à leur préparation. Seuls l'éthanol de titre approprié ou l'eau sont utilisés. Les extraits mous ont en général un résidu sec qui n'est pas inférieur à 70 pour cent m/m. Ils peuvent contenir des conservateurs antimicrobiens appropriés.

Les extraits secs sont des préparations solides, obtenues par évaporation du solvant ayant servi à leur production. Les extraits secs ont en général un résidu sec qui n'est pas inférieur à 95 pour cent m/m. Des substances inertes appropriées peuvent être ajoutées.

Les poudres de plantes sont obtenues à partir de plantes entières ou parties de plantes fragmentées ou coupées, utilisées en l'état, sous forme desséchée.

Selon le procédé de l'invention, les granules sont obtenus par montage par poudrage lorsque la substance végétale est sous la forme de poudre de plantes ou d'un extrait sec.

Le montage par poudrage est avantageusement effectué par pulvérisation alternée d'une solution alcoolique ou hydroalcoolique d'un liant, et de la substance végétale sous forme desséchée (poudre ou extrait sec).

Les granules sont obtenus par montage par pulvérisation lorsque la substance végétale est sous la forme d'un extrait mou ou fluide.

Dans le cas d'un extrait fluide, la couche active peut être enrobée d'une couche obtenue par pulvérisation d'une solution d'un liant. L'extrait fluide contient de préférence environ 30 à 40 % d'alcool.

Le procédé selon l'invention permet avantageusement de limiter la quantité de solvant organique utilisée. Au cours du procédé de l'invention, on utilise 5 à 25 % en poids de solvants organiques.

La taille des granules utilisés sera choisie en fonction du type d'extrait utilisé et en fonction du dosage souhaité.

Préférentiellement, la taille des neutres est comprise entre 200 et 900 µm, lorsque la substance végétale est sous forme desséchée (poudre ou extrait sec).

La taille des neutres est comprise entre 900 et 1250 µm, lorsque la substance végétale est sous forme liquide (extrait mou ou fluide).

Le pourcentage massique de substance végétale sous forme d'extrait fluide, utilisé dans le procédé de l'invention est avantageusement compris entre 15 et 50 % par rapport au poids des granules.

Le pourcentage massique de substance végétale sous forme desséchée (extrait, poudre) peut aller jusqu'à 75 % par rapport au poids des granules, il est de préférence compris entre 35 et 55 %.

Les granules selon l'invention sont préparés selon des techniques d'enrobage connus de l'homme du métier, de préférence en turbine ou en lit d'air fluidisé.

L'invention est illustrée de façon non limitative par les exemples suivants.

### Exemple 1 : Thé vert (extrait sec)

On prépare des granules de thé vert selon la succession d'étapes suivantes dans une turbine conventionnelle. Le thé vert est sous la forme d'un extrait sec.

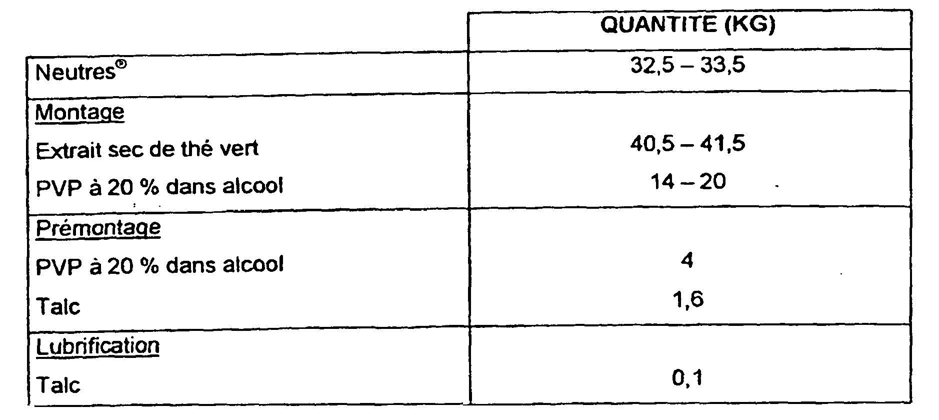

Les Neutres utilisées ont une granulométrie comprise entre 700 et 900 mm.

L'étape de montage du thé vert peut être effectuée en une ou plusieurs fois par pulvérisation alternée de l'extrait végétal et d'une solution de polyvinylpyrrolidone (PVP K30®) à 20 % dans l'éthanol.

Durant les étapes de montage, prémontage et lubrification, les granules sont tamisés respectivement à 1,0 - 1,18 mm, à 1,18 - 1,25 mm et de nouveau à 1,18 - 1,25 mm, puis séchés pendant 8 heures, respectivement à température ambiante et 30 °C.

On obtient des granules de formule suivante :

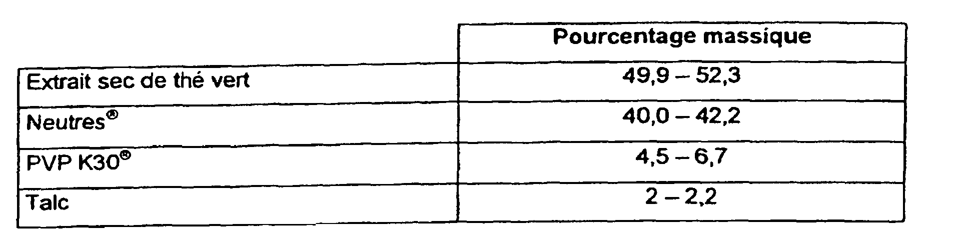

Leur teneur en eau est de l'ordre de 0,7 - 1,5 % en masse.

### Exemple 2 : Harpagophytum (extrait sec)

| **MATIERES PREMIERES** | **POURCENTAGE MASSIQUE** |
|---|---|
| Neutres | 39,9 |
| Extrait sec d'harpagophytum | 35,4 |
| PVP K30® | 2,6 |
| GLDB* | 2,2 |
| Alcool 95° | 19,4 |
| Talc | 0,5 |

| | |
|---|---|
| *GLDB : Gomme Laque Décirée Blanchie. | |

Les Neutres ont une granulométrie comprise entre 700 et 900 microns.

Les Neutres et l'extrait végétal sont pulvérisés avec une solution alcoolique de polyvinylpyrrolidone. Les granules sont tamisés et séchés. Au cours d'une deuxième étape, on applique une couche de gomme laque en utilisant toujours une solution alcoolique de polyvinylpyrrolidone.

Les granules sont à nouveau tamisés et séchés.

Enfin, les granules sont lubrifiés avec du talc.

### Exemple 3 : Harpagophytum (extrait fluide)

On prépare les granules de composition suivante, selon le procédé décrit ci-après.

| **MATIERES PREMIERES** | **POURCENTAGE MASSIQUE** |
|---|---|
| Extrait fluide d'harpagophytum | 18,5 |
| Neutres® | 67,4 |
| Saccharose crist. fin | 4,1 |
| Eau purifiée | 4,1 |
| Alcool | 5,2 |
| Talc | 0,7 |

Les Neutres sont introduits dans la cuve et l'extrait fluide est pulvérisé par fractions. Les granules sont calibrés par tamisage puis séchés sous lit d'air. On applique ensuite une solution de saccharose à 33 % dans un mélange éthanol/eau. Les granules sont à nouveau tamisés et séchés, puis lubrifiés avec du talc.

### Exemple 4 : Ginkgo biloba (extrait sec)

| **MATIERES PREMIERES** | **POURCENTAGE MASSIQUE** |
|---|---|
| Neutres® | 41,9 |
| Extrait sec de ginkgo biloba | 30,4 |
| PVP K 30® | 5,5 |
| Alcool 95° | 21,9 |
| Talc | 0,3 |

### Exemple 5 : Ginkgo biloba (extrait fluide)

| **MATIERES PREMIERES** | **POURCENTAGE MASSIQUE** |
|---|---|
| Extrait fluide de ginkgo biloba | 19,2 |
| Neutres® | 61,5 |
| PVP K 30® | 3,0 |
| Alcool à 95° | 12,3 |
| Talc | 4,0 |

Les Neutres sont introduits dans la cuve et l'extrait fluide est pulvérisé par fractions. Les granules sont calibrés par tamisage puis séchés sous lit d'air. On applique ensuite une solution de polyvinylpyrrolidone dans l'alcool. Les granules sont à nouveau tamisés et séchés, puis lubrifiés avec du talc.

### Exemple 6 : Valériane (extrait sec)

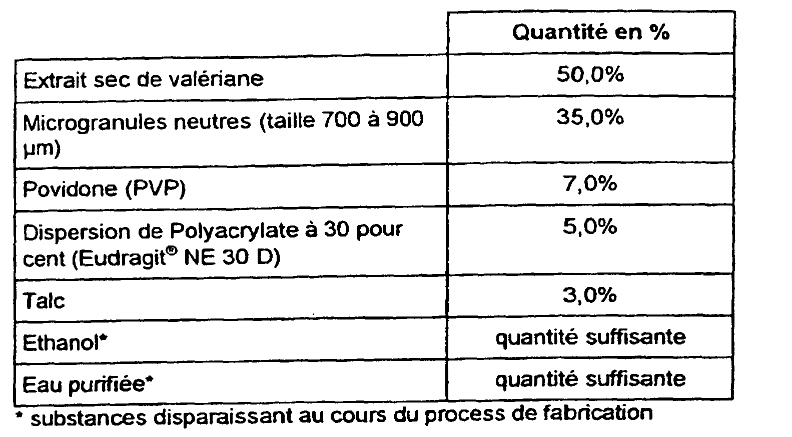

Le procédé de fabrication s'articule en 2 étapes principales:
- application de l'extrait sec de Valériane par poudrage sur des microgranules neutres, à l'aide d'un liant : solution alcoolique de PVP à 20%,
- enrobage des microgranules par un agent filmogène.

Le mode d'application de la matière active est discontinu, composé d'une succession de phases d'application alternées avec des phases de tamisage et de séchage durant lesquelles les microgranules sont placés sous un courant d'air chaud.

La phase d'enrobage se fait par pulvérisation de l'agent filmogène, sous forme de suspension à 30% dans l'eau, et d'une proportion de talc (prévenant la formation d'agrégats) suivie par une phase de tamisage et de séchage.

Enfin, les microgranules sont lubrifiés par addition de talc, réduisant l'électricité statique, facilitant le remplissage dans les gélules.

### Exemple 7 : Passiflore (extrait sec)

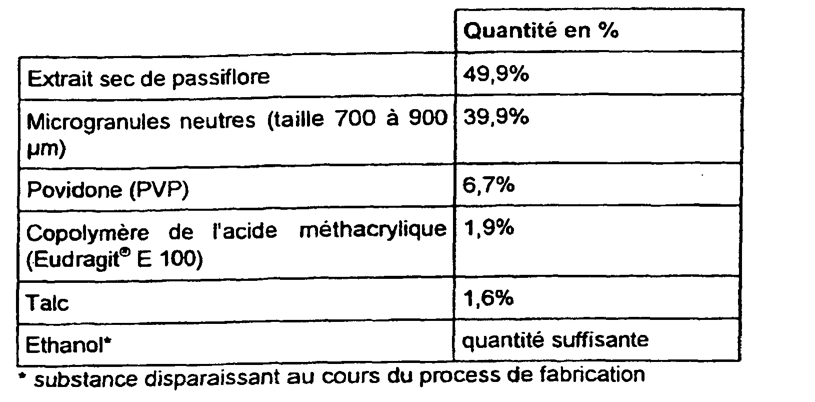

Le procédé de fabrication s'articule en 2 étapes principales:
- application de l'extrait sec de Passiflore par poudrage sur des microgranules neutres, à raide d'un liant : solution alcoolique de PVP à 20%,
- enrobage des microgranules par un agent filmogène.

Le mode d'application de la matière active est discontinu, composé d'une succession de phases d'application alternées avec des phases de tamisage et de séchage durant lesquelles les microgranules sont placés sous un courant d'air chaud.

La phase d'enrobage se fait par pulvérisation de l'agent filmogène, sous forme de solution alcoolique, et d'une proportion de talc (prévenant la formation d'agrégats) suivie par une phase de tamisage et de séchage.

Enfin, les microgranules sont lubrifiés par addition de talc, réduisant l'électricité statique, facilitant le remplissage dans les gélules.

### Exemple 8 : Vigne rouge (extrait sec)

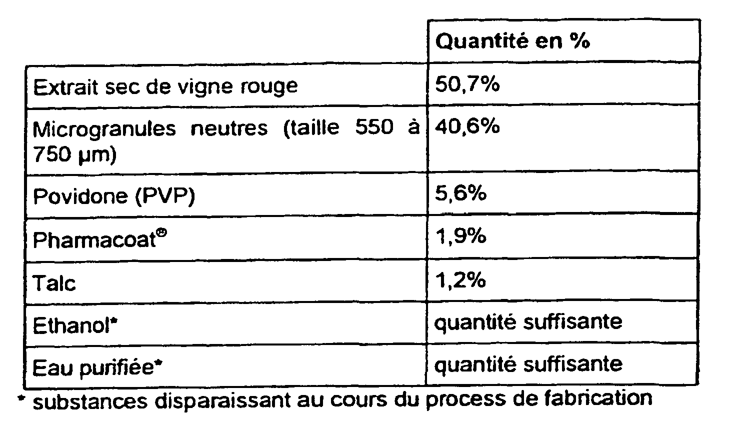

Le procédé de fabrication s'articule en 2 étapes principales:
- application de l'extrait sec de Vigne rouge par poudrage sur des microgranules neutres, à l'aide d'un liant : solution alcoolique de PVP à 20%.
- enrobage des microgranules par un agent filmogène.

Le mode d'application de la matière active est discontinu, composé d'une succession de phases d'application alternées avec des phases de tamisage et de séchage durant lesquelles les microgranules sont placés sous un courant d'air chaud.

La phase d'enrobage se fait par pulvérisation de l'agent filmogène, sous forme de solution hydroalcoolique, et d'une proportion de talc (prévenant la formation d'agrégats) suivie par une phase de tamisage et de séchage.

Enfin, les microgranules sont lubrifiés par addition de talc, réduisant l'électricité statique, facilitant le remplissage dans les gélules.

### Exemple 9 : Harpagohytum (extrait sec)

### • Formule :

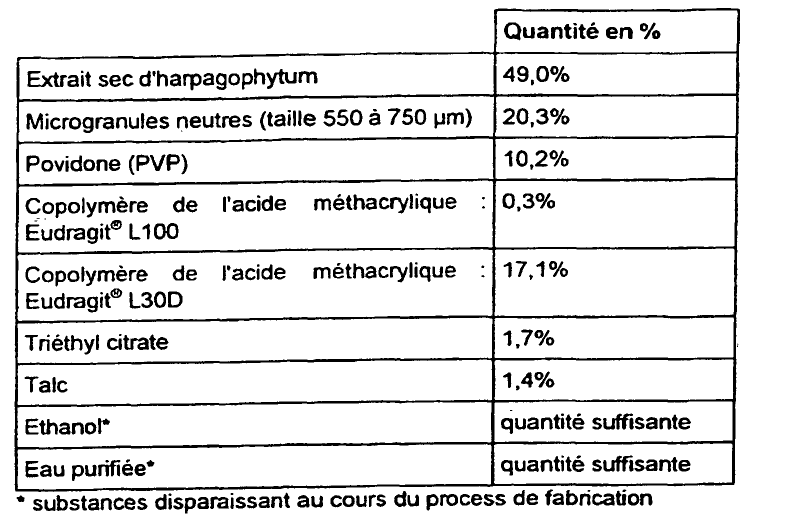

Le procédé de fabrication s'articule en 3 étapes principales:
Le procédé de fabrication s'articule en 3 étapes principales:
   - application de l'extrait sec d'Harpagophytum par poudrage sur des microgranules neutres, à l'aide d'un liant: solution alcoolique de PVP à 20%,
   - prémontage avec l'Eudragit® L100, pour préparer la surface des microgranules à l'enrobage
   - enrobage des microgranules par un agent filmogène : Eudragit® L30D additionné d'un plastifiant, assurant une gastroprotection.

Le mode d'application de la matière active est discontinu, composé d'une succession de phases d'application alternées avec des phases de tamisage et de séchage durant lesquelles les microgranules sont placés sous un courant d'air chaud.

L'application des agents filmogènes se fait par pulvérisation (Eudragit® L100, Eudragit® L30D, additionné du plastifiant : le triéthylcitrate) suivie par une phase de tamisage et de séchage.

Enfin, les microgranules sont lubrifiés par addition de talc, réduisant l'électricité statique, facilitant ainsi le remplissage dans les gélules.

### • Résultats des essais de gastroprotection sur 3 lots de microgranules d'harpagophytum

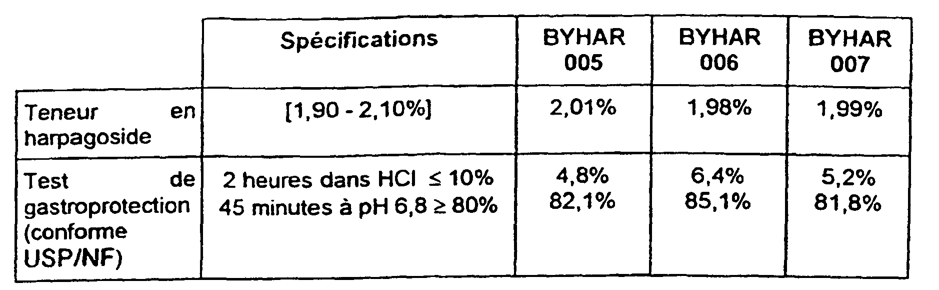

Les 3 lots de microgranules, fabriqués selon le procédé décrit ci-dessus, montrent des résultats conformes aux spécifications : ils sont gastroprotégés. L'harpagoside ne sera pas dégradé par les sucs gastriques.

### Exemple 10: Reproductibilité du procédé de fabrication.

### 1.1 Uniformité de dosage des gélules de microgranules.

### • 1^{ère} série de valeur

| Teneur en traceur | Lot BYVIG 002 | Lot BYVIG 003 | Lot BYVIG 0040 |
|---|---|---|---|
| Teneur théorique (%) | 5,44 % | 5,54 % | 5,52 % |
| Teneur mesurée (%) | 5.44% | 5.53% | 5.52% |

Les quantités de matières premières introduites sont retrouvées dans les produits finis : les écarts observés entre les valeurs théoriques (quantités introduites) et les valeurs mesurées (quantités retrouvées) sont non significatifs, ils sont inférieurs à 1 %.

### • 2^{nde} série de valeur

| Teneur en traceur | Lot TGIN 001 | Lot TGIN 002 | Lot TGIN 003 |
|---|---|---|---|
| Teneur théorique (%) | 5,0 % | 5.0% | 5.0% |
| Teneur mesurée (%) | 4.9% | 4,9 % | 4.9% |

Les trois lots étudiés, de même formulation, fabriqués selon le même mode opératoire, présentent des teneurs en matières premières actives semblables,

### 1.2 Uniformité de masse des gélules de microgranules

| Uniformité de masse | Normes | Lot 99.1.01 | Lot 99.1.04 | Lot 99.1.05 |
|---|---|---|---|---|
| Masse moyenne | 390 - 410 mg | 393 mg | 405 mg | 401 mg |
| Ecart de ±7,5 % | ≤ 2 gélules | Aucune gélule | 1 gélule | Aucune gélule |
| Ecart de ± 15% | Aucune gélule | Aucune gélule | Aucune Gélule | Aucune gélule |

Les trois lots testés satisfont au test d'uniformité de masse : les masses moyennes sont comprises dans les limites d'acceptation et moins de 2 gélules présente un écart de plus ou moins 7,5 % de la masse moyenne et aucune gélule de plus ou moins 15 %.

Le process de fabrication conduit à des gélules de qualité constante, il est parfaitement reproductible.

### 2. Stabilité des formules

Résultats de stabilité sur trois lots de gélules de microgranules de même formulation, après conservation pendant 6 mois en conditions normales de conservation et 6 mois en vieillissement accéléré :

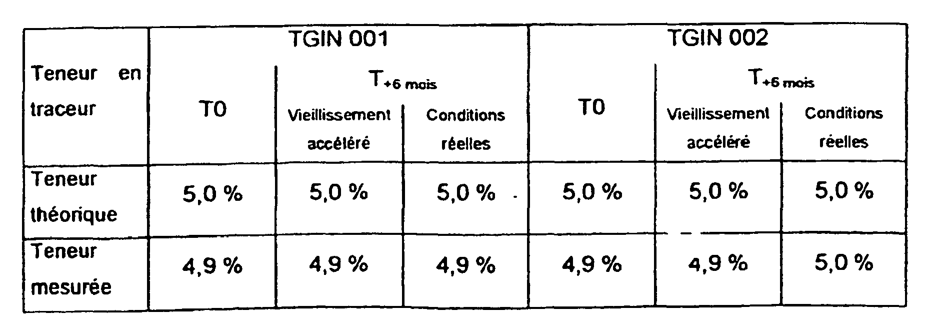

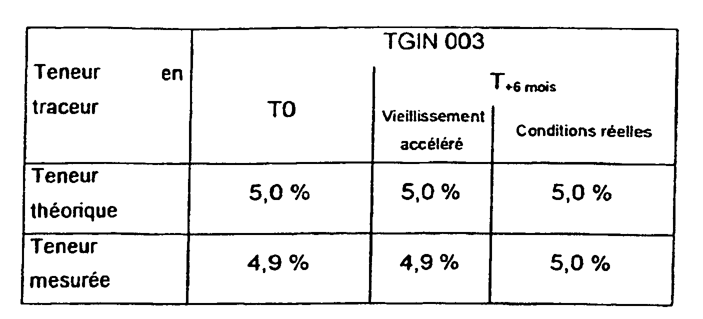

Les teneurs en traceurs des trois lots testés sont semblables aux teneurs théoriques et semblables à celles observées au temps TO : les trois lots sont parfaitement stables.

## Revendications

1. Granules contenant au moins une substance végétale comprennant chacun un noyau neutre de granulométrie comprise entre 0,2 et 4 mm enrobé d'une couche contenant la substance végétale associée à un excipient pharmaceutiquement acceptable **caractérisés en ce que** la couche contenant la substance végétale contient la polyvinylpyrrolidone comme liant.

2. Granules selon la revendication 1, **caractérisés en ce que** le noyau neutre est constitué d'une substance choisie parmi le sucre, l'amidon, le mannitol, le sorbitol, le xylitol, la cellulose, le talc et leurs mélanges.

3. Granules selon la revendication 1 ou 2, **caractérisés en ce que** le noyau neutre est constitué d'un coeur d'amidon/saccharose dans les proportions massiques 20/80 enrobé de 80 % en poids d'amidon.

4. Granules selon rune des revendications précédentes, **caractérisés en ce que** la couche contenant la substance végétale est enrobée d'une couche externe destinée à masquer le goût et/ou l'odeur de la substance végétale, à retarder sa libération ou à contrôler sa libération.

5. Granules selon la revendication 4, **caractérisés en ce que** la couche externe est destinée à contrôler la libération de la substance végétale et contient de la gomme laque, de la PVP, un copolymère de l'acide méthacrylique ou une dispersion colloïdale d'éthylcellulose avec un plastifiant

6. Granules selon la revendication 4, **caractérisés en ce que** la couche externe est destinée à retarder la libération de la substance végétale et contient un copolymère de Facide méthacrylique, de la gomme laque ou une dispersion colloïdale d'éthylcellulose avec un plastifiant.

7. Granules selon la revendication 4, **caractérisés en ce que** la couche externe est destinées à masquer le goût et/ou l'odeur de la substance végétale et contient une dispersion de polyacrylate, un copolymère de l'acide méthacrylique ou de l'hydroxypropylméthylcellulose.

8. Granules selon l'une des revendications précédentes, **caractérisés en ce que** la substance végétale est choisie parmi l'ail, l'échinacée, le ginkgo biloba, le ginseng, l'harpagophytum, le kawa kawa, le millepertuis, le thé vert, la valériane, la vigne rouge, l'artichaut, l'aubépine, la bardane, le bouleau, la bourdaine, le cassis, le chardon Marie, le fucus, l'hamamelis, le marron d'Inde, la mélisse, l'orthosiphon, la passiflore, le pissenlit, la prêle, la reine des prés, la sauge, la spiruline, le boldo, l'ortie, le palmier d'Amérique du Nord et leurs mélanges.

9. Granules selon l'une des revendications précédentes **caractérisés en ce que** la teneur en substance végétale est comprise entre 0,1 mg/g et 750 mg/g.

10. Procédé de préparation des granules selon l'une des revendications précédentes, **caractérisé en ce que** la substance végétale montée sur les noyaux neutres est sous la forme d'une poudre de plantes ou d'un extrait sec, mou ou fluide.

11. Procédé de préparation selon la revendication 10, **caractérisé en ce que** les granules sont obtenus par montage par poudrage lorsque la substance végétale est sous forme sèche, c'est-à-dire sous forme de poudre de plantes ou d'extrait sec.

12. Procédé selon la revendication 10 , **caractérisé en ce que** les granules sont obtenus par montage par pulvérisation lorsque la substance végétale est sous la forme d'un extrait mou ou fluide.

13. Procédé selon la revendication 12, **caractérisé en ce que** l'extrait fluide contient de 30 à 40 % d'alcool.

14. Procédé selon l'une des revendications 10 à 13, **caractérisé en ce qu'**on utilise 5 à 25 % en poids de solvants organiques.

15. Procédé selon l'une des revendications 10, 11 et 14, **caractérisé en ce que** la taille des neutres est comprise entre 200 et 900 µm, lorsque la substance végétale est sous forme desséchée.

16. Procédé selon l'une des revendications 10 et 12 à 14, **caractérisé en ce que** la taille des neutres est comprise entre 900 et 1250 µm, lorsque la substance végétale est sous la forme d'un extrait mou ou fluide.

17. Procédé selon l'une des revendications 10, 12 à 14 et 16, **caractérisé en ce que** le pourcentage massique de substance végétale sous forme d'extrait fluide utilisée est compris entre 15 et 50 % par rapport au poids des granules.

18. Procédé selon l'une des revendications 10, 11, 14 et 15, **caractérisé en ce que** le pourcentage massique de substance végétale sous forme desséchée utilisée peut aller jusqu'à 75 % par rapport au poids des granules.

19. Procédé selon l'une des revendications précédentes, **caractérisé en ce que** les granules sont préparés en turbine ou en lit d'air fluidisé.

## Patentansprüche

1. Granalien, die mindestens eine pflanzliche Substanz enthalten und jeweils einen neutralen Kern mit einer Korngröße zwischen 0,2 und 4 mm einschließlich umfassen, der mit einer Schicht umhüllt ist, welche die pflanzliche Substanz assoziiert mit einem pharmazeutisch annehmbaren Hilfsstoff umfasst, **dadurch gekennzeichnet, dass** die Schicht, welche die pflanzliche Substanz enthält, Polyvinylpyrrolidon als Bindemittel enthält.

2. Granalien nach Anspruch 1, **dadurch gekennzeichnet, dass** der neutrale Kern aus einer Substanz besteht, die aus Zucker, Stärke, Mannit, Sorbit, Xylit, Cellulose, Talkum und deren Mischungen ausgewählt ist.

3. Granalien nach Anspruch 1 oder 2, **dadurch gekennzeichnet, dass** der neutrale Kern aus .einem Stärke/Saccharose-Herzstück in den Massenanteilen 20/80 besteht, das von 80 Gew.-% Stärke umhüllt ist.

4. Granalien nach einem der vorangehenden Ansprüche, **dadurch gekennzeichnet, dass** die Schicht, welche die pflanzliche Substanz enthält, von einer äußeren Schicht umhüllt ist, die dazu bestimmt ist, den Geschmack und/oder den Geruch der pflanzlichen Substanz zu maskieren, ihre Freisetzung zu verzögern oder ihre Freisetzung zu steuern.

5. Granalien nach Anspruch 4, **dadurch gekennzeichnet, dass** die äußere Schicht dazu bestimmt ist, die Freisetzung der pflanzlichen Substanz zu steuern, und Naturharzgummi, PVP, ein Copolymer von Methacrylsäure oder eine kolloidale Dispersion von Ethylcellulose mit einem Weichmacher enthält.

6. Granalien nach Anspruch 4, **dadurch gekennzeichnet, dass** die äußere Schicht dazu bestimmt ist, die Freisetzung der pflanzlichen Substanz zu verzögern, und ein Copolymer von Methacrylsäure, Naturharzgummi oder eine kolloidale Dispersion von Ethylcellulose mit einem Weichmacher enthält.

7. Granalien nach Anspruch 4, **dadurch gekennzeichnet, dass** die äußere Schicht dazu bestimmt ist, den Geschmack und/oder den Geruch der pflanzlichen Substanz zu maskieren, und eine Polyacrylat-Dispersion, ein Copolymer von Methacrylsäure oder Hydroxypropylmethylcellulose enthält.

8. Granalien nach einem der vorangehenden Ansprüche, **dadurch gekennzeichnet, dass** die pflanzliche Substanz ausgewählt ist aus Knoblauch, Echinacea, Gingko biloba, Ginseng, Harpagophytum, Kawa-Kawa, Johanniskraut, grünem Tee, Baldrian, roter Weinrebe, Artischocke, Weißdorn, Klette, Birke, Faulbaum, Schwarzer Johannisbeere, Mariendistel, Ledertang, Hamamelis, Rosskastanie, Melisse, Orthosiphon, Passionsblume, Löwenzahn, Schachtelhalm, Wiesenkönigin, Salbei, Spirulina platensis, Boldo, Brennessel, nordamerikanischer Palme und deren Mischungen.

9. Granalien nach einem der vorangehenden Ansprüche, **dadurch gekennzeichnet, dass** der Gehalt an pflanzlicher Substanz zwischen 0,1 mg/g und 750 mg/g einschließlich liegt.

10. Verfahren zur Herstellung von Granalien nach einem der vorangehenden Ansprüche, **dadurch gekennzeichnet, dass** die pflanzliche Substanz, die auf den neutralen Kernen angebracht wird, in Form eines Pulvers von Pflanzen oder eines trockenen, weichen oder dünnflüssigen Extrakts vorliegt.

11. Verfahren zur Herstellung nach Anspruch 10, **dadurch gekennzeichnet, dass** die Granalien durch Aufbringen durch Bepudern erhalten werden, wenn die pflanzliche Substanz in trockener Form vorliegt, d.h. in Form von Pulver von Pflanzen oder von trockenem Extrakt.

12. Verfahren nach Anspruch 10, **dadurch gekennzeichnet, dass** die Granalien durch Aufbringen mittels Zerstäubung erhalten werden, wenn die pflanzliche Substanz in Form eines weichen oder dünnflüssigen Extrakts vorliegt.

13. Verfahren nach Anspruch 12, **dadurch gekennzeichnet, dass** der dünnflüssige Extrakt 30 bis 40 % Alkohol enthält.

14. Verfahren nach einem der Ansprüche 10 bis 13, **dadurch gekennzeichnet, dass** man 5 bis 25 Gew.-% organische Lösungsmittel verwendet.

15. Verfahren nach einem der Ansprüche 10, 11 und 14, **dadurch gekennzeichnet, dass** die Größe der neutralen Kerne zwischen 200 und 900 µm einschließlich liegt, wenn die pflanzliche Substanz in getrockneter Form vorliegt.

16. Verfahren nach einem der Ansprüche 10 und 12 bis 14, **dadurch gekennzeichnet, dass** die Größe der neutralen Kerne zwischen 900 und 1250 µm einschließlich liegt, wenn die pflanzliche Substanz in Form eines weichen oder dünnflüssigen Extrakts vorliegt.

17. Verfahren nach einem der Ansprüche 10, 12 bis 14 und 16, **dadurch gekennzeichnet, dass** der Gewichtsprozentsatz an verwendeter pflanzlicher Substanz in Form von flüssigem Extrakt zwischen 15 und 50 % einschließlich liegt, bezogen auf das Gewicht der Granalien.

18. Verfahren nach einem der Ansprüche 10, 11, 14 und 15, **dadurch gekennzeichnet, dass** der Gewichtsprozentsatz der verwendeten pflanzlichen Substanz in getrockneter Form bis zu 75 %, bezogen auf das Gewicht der Granalien, gehen kann.

19. Verfahren nach einem der vorangehenden Ansprüche, **dadurch gekennzeichnet, dass** die Granalien in einer Zentrifuge oder in einem durch Luft fluidisierten Bett hergestellt werden.

## Claims

1. Granules containing at least one plant substance, each comprising a neutral core having a particle size of between 0.2 and 4 mm coated with a layer containing the plant substance combined with a pharmaceutically acceptable excipient, **characterized in that** the layer containing the plant substance contains polyvinylpyrrolidone as binder.

2. Granules according to Claim 1, **characterized in that** the neutral core consists of a substance chosen from sugar, starch, mannitol, sorbitol, xylitol, cellulose, talc and mixtures thereof.

3. Granules according to Claim 1 or 2, **characterized in that** the neutral core consists of a starch/sucrose core in 20/80 mass ratios which is coated with 80% by weight of starch.

4. Granules according to one of the preceding claims, **characterized in that** the layer containing the plant substance is coated with an outer layer intended to mask the taste and/or the odour of the plant substance, to delay its release or to control its release.

5. Granules according to Claim 4, **characterized in that** the outer layer is intended to control the release of the plant substance and contains lac gum, PVP, a copolymer of methacrylic acid or a colloidal dispersion of ethyl cellulose with a plasticizer.

6. Granules according to Claim 4, **characterized in that** the outer layer is intended to delay the release of the plant substance and contains a copolymer of methacrylic acid, lac gum or a colloidal dispersion of ethyl cellulose with a plasticizer.

7. Granules according to Claim 4, **characterized in that** the outer layer is intended to mask the taste and/or the odour of the plant substance and contains a polyacrylate dispersion, a copolymer of methacrylic acid or hydroxypropylmethylcellulose.

8. Granules according to one of the preceding claims, **characterized in that** the plant substance is chosen from garlic, Echinacea, Ginkgo biloba, ginseng, Harpagophytum, kawa St.-John's-wort, green tea, valerian, grape vine, artichoke, hawthorn, burdock, birch, alder buckthorn, blackcurrant, blessed thistle, Fucus, Hamamelis, horse chestnut, balm, Orthosiphon, passion flower, dandelion, horsetail, meadowsweet, sage, spirulina, boldo, nettle, North American palm and mixtures thereof.

9. Granules according to one of the preceding claims, **characterized in that** the content of plant substance is between 0.1 mg/g and 750 mg/g.

10. Process for the preparation of granules according to one of the preceding claims, **characterized in that** the plant substance coated onto the neutral cores is in the form of a plant powder or of a dry, soft or fluid extract.

11. Process of preparation according to Claim 10, **characterized in that** the granules are obtained by powder-coating when the plant substance is in dry form, that is to say in the form of plant powder or of a dry extract.

12. Process according to Claim 10, **characterized in that** the granules are obtained by spray-coating when the plant substance is in the form of a soft or fluid extract.

13. Process according to Claim 12, **characterized in that** the fluid extract contains from 30 to 40% alcohol.

14. Process according to one of Claims 10 to 13, **characterized in that** 5 to 25% by weight of organic solvents are used.

15. Process according to one of Claims 10, 11 and 14, **characterized in that** the size of the Neutres is between 200 and 900 *µ*m, when the plant substance is in desiccated form.

16. Process according to one of Claims 10 and 12 to 14, **characterized in that** the size of the Neutres is between 900 and 1250 µm, when the plant substance is in the form of a soft or fluid extract.

17. Process according to one of Claims 10, 12 to 14 and 16, **characterized in that** the percentage by mass of plant substance in the form of fluid extract used is between 15 and 50% relative to the weight of the granules.

18. Process according to one of Claims 10, 11, 14 and 15, **characterized in that** the percentage by mass of plant substance in desiccated form used may be as high as 75% relative to the weight of the granules.

19. Process according to one of the preceding claims, **characterized in that** the granules are prepared in a turbine or in a fluidized air bed.
